(19) **Europäisches Patentamt / European Patent Office / Office européen des brevets**

(11) **EP 4 368 236 A1**

(12) **EUROPEAN PATENT APPLICATION**

(43) Date of publication:
**15.05.2024 Bulletin 2024/20**

(21) Application number: **22386080.0**

(22) Date of filing: **11.11.2022**

(51) International Patent Classification (IPC):
**A61N 1/04** (2006.01)    **A61N 1/30** (2006.01)

(52) Cooperative Patent Classification (CPC):
**A61N 1/0428; A61N 1/30;** A61N 1/306

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Cambridge Enterprise Limited**
**Cambridge, Cambridgeshire CB2 1TN (GB)**

(72) Inventor: **The designation of the inventor has not yet been filed**

(74) Representative: **J A Kemp LLP**
**80 Turnmill Street**
**London EC1M 5QU (GB)**

(54) **IONTOPHORETIC DRUG DELIVERY DEVICE**

(57) The present invention relates to a drug delivery device, a method to transport a charged species, a method to manufacture a drug delivery device, and a method to generate a charged fluid. A drug delivery device is configured to deliver a drug to a drug delivery site in a bodily tissue, the drug delivery device comprising a cavity configured to continuously receive a charged fluid, wherein the drug delivery device is configured to receive a charged particle comprising a drug in a space adjacent to the cavity and drive the charged particle from the space to the drug delivery site, the charged particle being driven by the action of an electric field generated by the charged fluid in the cavity.

FIG. 7

EP 4 368 236 A1

## Description

### Field of the invention

The present invention relates to a drug delivery device, a method to transport a charged species, a method to manufacture a drug delivery device, and a method to generate a charged fluid.

### Background

With more than 1 1,000 new cases each year in the UK, brain tumours are a common disease which significantly impacts the life of affected patients. On average, only 12% of patients survive for five or more years after the diagnosis. Depending on each individual case, brain tumours are treated by a combination of chemotherapy, radiotherapy and, if possible, surgical resection. A fundamental limitation for the success of chemotherapy is the blood-brain-barrier which plays a pivotal role in protecting the central nervous system from toxic substances but also significantly reduces the amount of cancer drugs which can be delivered into a tumour systemically. Additionally, bad vascularisation of solid brain tumours further complicates delivery of therapeutically relevant drug doses. Off-target toxicity of many common chemotherapeutics places strong constraints on the amount of drugs which can be safely systemically administered.

Convection enhanced delivery is a technique of pumping a drug fluid directly into a treatment site of a patient, such as a tumour. The quantity of a drug that can be safely delivered by convection enhanced delivery is limited by the local pressure build-up that inevitably occurs at the delivery site when the fluid is delivered. This technique also suffers from the problem of drug reflux.

Iontophoresis is the use of electric fields for the delivery of charged species. Iontophoresis is applicable in the field of drug delivery and is distinguished from convection-enhanced drug delivery in that iontophoresis is capable of delivering only a drug molecule or particle to the site of interest, without also needing to deliver the bulk fluid in which the drug is dissolved or suspended. Thus, delivery of drugs by iontophoresis can achieve a high drug concentration at a drug delivery site without drastically increasing local pressure at the site.

While iontophoresis has been used since the 1920s, implantable iontophoretic devices are relatively new. Notable work is published in the following documents, all of which are incorporated by reference herein in their entirety:

(1) EP 2 401 027 B1.
(2) J. D. Byrne et al. 'Local iontophoretic administration of cytotoxic therapies to solid tumors'. In: Science Translational Medicine 7.273 (Feb. 2015), 273ra14-273ra14; doi: 10.1126/scitranslmed.3009951.

(3) J. D. Byrne et al. 'Iontophoretic device delivery for the localized treatment of pancreatic ductal adenocarcinoma'. In: Proceedings of the National Academy of Sciences 113.8 (Feb. 2016), pp. 2200-2205; doi: 10.1073/pnas.1600421113.
(4) J. D. Byrne, J. J. Yeh and J. M. DeSimone. 'Use of iontophoresis for the treatment of cancer'. In: Journal of Controlled Release 284 (Aug. 2018), pp. 144-151; doi: 10.1016/j.jconrel.2018.06.020.
(5) T. A. Sjöström et al. 'A Decade of Iontronic Delivery Devices'. In: Advanced Materials Technologies 3.5 (Mar. 2018), p. 1700360; doi: 10.1002/admt.201700360.
(6) A. Williamson et al. 'Controlling epileptiform activity with organic electronic ion pumps'. In: Advanced Materials 27.20 (2015), pp. 3138-3144; doi: 10.1002/adma.201500482.
(7) L. Waldherr et al. 'Targeted Chemotherapy of Glioblastoma Spheroids with an Iontronic Pump'. In: Advanced Materials Technologies (Apr. 2021), p. 2001302; doi: 10.1002/admt.202001302.

A fundamental challenge for iontophoresis is the creation of electric fields for extended periods of time because at perfectly blocking electrodes (i.e. electrodes where no electrochemical reactions are occurring), electrical double layers quickly form on the electrode surface which screen the electric field. As a result, with blocking electrodes drugs cannot be delivered. In traditional iontophoresis therefore, high voltages are applied which cause hydrolysis of water. While this reaction allows charge transfer, large amounts of corrosive chlorine gas form at the anode and hydrogen gas at the cathode, which can damage the device and can cause harm to the patient. Furthermore, the gas builds up in the delivery device and significantly reduces efficiency.

An alternative approach is using a solid electrode material which can be oxidised/reduced, such as Ag/AgCl or PEDOT:PSS. $Ag^+$ however is toxic to the patient and both Ag/AgCl and PEDOT:PSS electrodes are depleted during use, and therefore limit how long the device can work.

Another limitation of existing drug delivery devices utilising an electrode is that the drug comes in contact with the electrode and can therefore itself undergo a redox reaction which might render it harmful. Depending on the applied voltage, any molecule coming into contact with the working electrode may undergo some form of electrochemical reaction. As a consequence, traditional iontophoretic devices will chemically change the drug molecules over time which can render them useless and/or harmful. The possible drug reactions will be very likely a severe issue when trying to get regulatory approval for a medical device.

Iontophoretic devices which have been published in literature may be grouped into two categories: traditional iontophoretic devices; and organic electronic ion pumps (OEIPs).

*Traditional iontophoretic devices*

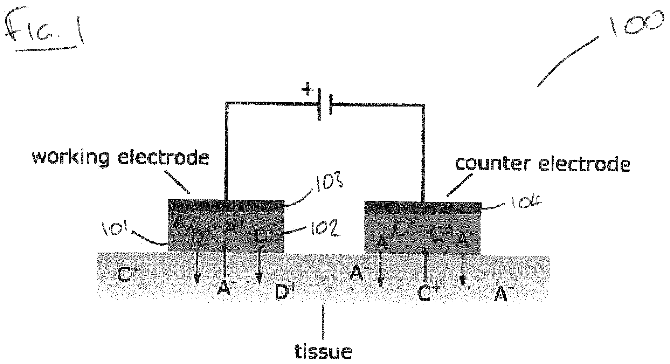

Traditional iontophoretic drug delivery devices have been used for transdermal delivery (i.e. delivery across the skin of a patient) of drugs. An example of a traditional iontophoretic device is shown schematically in Figure 1.

The traditional iontophoretic device 100 comprises a reservoir 101 containing the drug molecule 102 and the working electrode 103. The reservoir is either open or sealed off using a membrane. The device 100 also comprises a counter electrode 104 placed elsewhere on the patient, far away from the working electrode 103. In the example of Figure 1, an electric current (e.g. DC current) applied between the electrodes 103, 104 causes a positively-charged drug ($D^+$) to be driven into the tissue (skin) of the patient in the vicinity of the anode 103 (working electrode). Anions $A^-$ in the skin of the patient (such as $Cl^-$ ions) are drawn to the anode 103, and cations $C^+$ are drawn to the cathode 104 (counter electrode). These traditional devices are based on hydrolysis, i.e. the water in which the drug is dissolved is split on the electrodes as shown in the following electrochemical half-equations.

$$\text{Anode:} \qquad 2Cl^- \rightarrow Cl_2 \uparrow + 2e^-$$

$$\text{Cathode:} \qquad 2H_2O + 2e^- \rightarrow 20H^- + H_2 \uparrow$$

Due to these reactions, large amounts of corrosive chlorine gas form at the anode and hydrogen gas at the cathode. The gas formation on the electrodes causes significant problems as gas bubbles will accumulate in the device rendering the device inoperable after a while. Also, at both electrodes very corrosive species are formed: at the anode chlorine gas and at the cathode $OH^-$, which will damage the device. This fundamentally limits the long term usefulness of the known devices.

Hydrolysis generally happens at both the working electrode (the anode: producing $Cl_2$ gas) and the counter electrode (the cathode: producing $H^2$ gas). In some arrangements, the working electrode can be the cathode, it depends on the charge of the drug which needs to be delivered. 'Working electrode' is used herein as the name for the electrode where the drug is repelled from.

*Organic electronic ion pumps (OEIPs)*

OEIPs have emerged more recently than traditional iontophoretic devices, and are very similar to the traditional iontophoretic devices. The only difference to traditional iontophoretic devices is that OEIPs use a solid film of the conducting polymer PEDOT:PSS as working electrode (anode). PEDOT:PSS can be oxidised and therefore allows to drive the device for a short time until the PEDOT:PSS film is depleted. Then, drug delivery stops. An advantage of this approach is that no gases are formed but a disadvantage is that the devices can be operated only for a relatively short time due to depletion of the electrodes. OEIPs are usually used for delivery of small amounts of molecules directly into cells over a short time period and thus are not suitable for many applications such as cancer drug delivery.

Summary of the invention

In view of these problems, there is a need for devices and methods capable of safely delivering high concentrations of drugs to a delivery site, over long periods of time, without being subject to problems of off-target toxicity. Such devices and methods would be particularly useful in the treatment of cancerous tumours in the brain and elsewhere, and of various other diseases and conditions.

A first aspect of the present invention is a drug delivery device configured to deliver a drug to a drug delivery site in a bodily tissue, said drug delivery device comprising a cavity configured to continuously receive a charged fluid, wherein the drug delivery device is configured to: receive a charged particle comprising a drug in a space adjacent to the cavity; and drive the charged particle from the space to the drug delivery site, the charged particle being driven by the action of an electric field generated by the charged fluid in the cavity.

The charged fluid received in the cavity of the drug delivery device forms effectively a "fluid electrode", which generates an electric field to interact with the charged particle comprising the drug in the space adjacent to the cavity. The charged fluid is continuously replenished and thus not subject to degradation in the way that a solid electrode directly in contact with a drug solution would be. An electric field may therefore be maintained for a long time, enabling long-term and stable delivery of the drug. The drug delivery device operates on the principles of iontophoresis, that is, using electric fields to drive the movement of the drug. The device is therefore capable of supplying a high concentration of the drug to the site of interest without causing local over-pressures. Furthermore, by providing the drug in a space adjacent to the cavity, rather than in the cavity with the charged fluid itself, detrimental reactions of the drug with other substances (such as the charged fluid, or any solid electrode that may be present inside the cavity) are prevented.

The charged particle comprising the drug may be the drug itself (i.e. a charged atom or molecule that is the drug to be delivered). Alternatively, the charged particle comprising the drug may be a particle (such as a nanoparticle) carrying the drug and other components. The charged particle comprising the drug may be a constituent of a drug fluid comprising other substances such as a solvent.

The cavity may be a channel configured to receive a continuous flow of the charged fluid. The charged fluid is a fluid (e.g. a liquid) that comprises a charged component, and thereby possesses as a whole a net or overall charge, which is either positive or negative in polarity.

Preferably, the space adjacent to the cavity is a drug channel configured to continuously receive the charged particle comprising the drug. In such an embodiment, the period of time over which the drug delivery device can deliver a drug to the drug delivery site of the patient is not limited by the size of the device itself. Rather, it is limited only by the length of time over which the drug and the charged fluid are supplied to the device. This therefore enables very long-term, theoretically endless, delivery of the drug to the patient.

Preferably, the drug delivery device comprises an electrode (i.e. a working electrode) in fluid contact with the cavity. The electrode in fluid contact with the cavity may be a solid electrode. The electrode is configured to provide a charge to a fluid so as to generate the charged fluid. That is, the charged fluid may be generated at the electrode by a charge-transfer interaction between the electrode and a feed fluid that initially does not carry an overall charge. In this embodiment, the position of the electrode relative to the cavity and the space adjacent to the cavity is not particularly limited. The electrode may be provided inside the cavity, and an uncharged fluid continuously supplied to the cavity, such that the charged fluid is continuously generated inside the drug delivery device. Additionally or alternatively, the electrode may be provided at a position upstream of the cavity, such that the fluid gains a charge prior to entry to the cavity, and the charged fluid is continuously supplied to the drug delivery device. In the case where the drug delivery device is configured to be implanted or partially implanted in a patient tissue, the electrode may be positioned inside the bodily tissue during use or outside the bodily tissue during use.

In use of the drug delivery device, a counter-electrode may be positioned elsewhere on the body of the subject to whom the drug is to be delivered, such that a voltage may be applied between the working electrode and the counter-electrode to drive the drug delivery.

Preferably, the drug delivery device comprises a first membrane between the cavity and the space adjacent to the cavity. The first membrane may selectively prevent any bulk fluid (such as solvent) from passing either way between the cavity and the space adjacent to the cavity. The first membrane may therefore advantageously prevent mixing of fluids between the different cavities which could otherwise lead to inefficiency or inefficacy of the device.

The first membrane may be configured to selectively allow a species having a charge of opposite polarity to that of the charged particle comprising the drug to pass into the cavity from the space adjacent to the cavity. This may enhance the driving of the drug to the drug delivery site by the electric field generated by the charged fluid in the cavity. The first membrane may additionally or alternatively be configured to selectively allow a species having a charge of the same polarity as that of the charged particle comprising the drug to pass into the cavity from the space adjacent to the cavity.

The first membrane may be a non-selective membrane such as a porous membrane, which permits particles of either charge polarity (or no charge) to pass through it. Alternatively, the first membrane may be a selective membrane. The first membrane may be charge-selective in the sense that only particles of a certain charge polarity are permitted to pass through. The first membrane may be, for instance, an ion exchange membrane. When the first membrane is a selective membrane, such as an ion exchange membrane, the first membrane may prevent passage therethrough of certain species. For instance, the selectivity of the first membrane may prevent the charged particle comprising the drug from entering the cavity, and/or prevent charged species within the charged fluid from entering the space adjacent to the cavity. This selectivity is advantageous because it can prevent detrimental reactions that would otherwise occur, such as a reaction between the drug and the charged fluid, or between the drug and an electrode inside the cavity.

Preferably, the drug delivery device comprises a second membrane between the space adjacent to the cavity and the drug delivery site. The second membrane is configured to selectively allow the charged particle comprising the drug to pass to the drug delivery site from the space adjacent to the cavity. The action of the electric field generated by the charged fluid in the cavity drives the charged particle comprising the drug across the second membrane towards the drug delivery site. The second membrane may prevent passage of a bulk drug fluid from the space adjacent to the cavity to the drug delivery site. That is, when the space adjacent to the cavity contains a drug fluid that supports the charged particle comprising the drug (e.g. a solvent in which the a drug is dissolved, or a liquid in which the charged particles comprising the drug are suspended), the second membrane may selectively allow passage of only the charged particle comprising the drug to the drug delivery site, while retaining the remaining drug fluid in the space adjacent to the cavity. The second membrane thus enables iontophoretic delivery of the charged particle comprising the drug to the drug delivery site, while preventing the potentially harmful transfer of non-therapeutic fluid to the drug delivery site. The second membrane may be a non-selective membrane such as a porous membrane, or may be a selective membrane such as an ion exchange membrane.

A second aspect of the invention is a method to transport a charged species, said method comprising: continuously receiving a fluid in a cavity of a transport device, said fluid having or being provided with an electric charge so as to generate an electric field; receiving a charged species in a space adjacent to the cavity; and transporting the charged species away from the space, the charged species being transported by the action of said electric field.

Optionally, the charged species is transported in a direction away from the cavity in addition to being transported away from the space adjacent to the cavity.

A third aspect of the invention is a method of manufacturing a drug delivery device, said method comprising: providing a component having a cavity therein, the cavity configured to continuously receive a charged fluid; providing a first membrane between the cavity and a space adjacent to the cavity; and providing a second membrane in contact with the space adjacent to the cavity, the second membrane being configured to selectively allow a particle comprising a drug to pass out of the space adjacent to the cavity in a direction away from the cavity.

A fourth aspect of the invention is a drug delivery device comprising a fluid for delivering a drug to a drug delivery site, the fluid configured to: gain a charge from an electrode; and when charged, generate an electric field to drive a charged particle in a space adjacent to the fluid in a direction away from the fluid, the charged particle comprising the drug.

The fluid in the drug delivery device is preferably configured to gain a charge from a solid electrode. That is, the fluid includes a species that is able to undergo a charge transfer interaction with the electrode, thereby imparting to the fluid a net positive or negative charge. For instance, the fluid may have a composition including a species that is readily capable of being oxidised or reduced at the electrode, thereby facilitating ionic-electric charge transfer between the electrode and the fluid. Drug delivery by the drug delivery device is driven by this charge transfer interaction. The drug delivery device operates on the principles of iontophoresis, and is therefore capable of supplying a high concentration of the drug to the site of interest without causing local over-pressures. Furthermore, by providing the drug in a space adjacent to the cavity, rather than in the cavity with the charged fluid itself, detrimental reactions of the drug with other substances (such as the charged fluid, or any solid electrode that may be present inside the cavity) are prevented. The cavity may be channel configured to receive a continuous flow of the fluid.

Preferably, the fluid is configured to gain the charge from the electrode without causing significant generation of a gas at the electrode. More preferably, the fluid is configured to gain the charge from the electrode without causing any generation of gas at the electrode. Traditional iontophoretic devices whose driving mechanism is a gas-generating reaction such as hydrolysis, are subject to the problems of poor longevity of the device due to release of (possibly corrosive) gases or other corrosive species, and harm to the patient due to the release of such gases inside the body. The drug delivery device of the present aspect does not rely on such gas-generating reactions to drive the iontophoretic delivery of drugs and thus is useful for safe long-term drug delivery to a patient tissue.

Optionally, the drug delivery device comprises the electrode that is configured to provide the fluid with the charge.

In use, a counter-electrode may be positioned elsewhere on the body of the subject to whom the drug is to be delivered, such that a voltage may be applied between the working electrode and the counter-electrode to drive the drug delivery.

Preferably, the drug delivery device is configured to continuously receive a flow of the fluid, wherein a flow path of the fluid contacts the electrode such that the fluid gains the charge when it passes the electrode. The fluid is thus continuously replenished and continuously provided with a charge by the electrode, and thus not subject to degradation in the way that a solid electrode directly in contact with a drug solution would be. An electric field may therefore be maintained for a long time, enabling long-term and stable delivery of the drug. Because the charge transfer interaction undergone by the fluid with the electrode does not cause significant (or any) generation of a gas, the continuous provision of a charge to the fluid does not cause detrimental gas build-up over time which would otherwise damage the device and/or cause harm to a patient if occurring inside the tissue.

A fifth aspect of the present invention is a method to transport a charged species, said method comprising: providing a fluid with an electric charge; positioning the charged species in a space adjacent to the fluid provided with the electric charge; and driving the charged species in a direction away from the fluid, by action of an electric field generated by the fluid.

A sixth aspect of the present invention is a drug delivery device comprising an electrode and a channel, the channel being in contact with the electrode and configured to receive a fluid, wherein: the electrode is formed of a double-sided piece of electrode material; and the channel is shaped so as to be in contact with two sides of the piece of electrode material.

The drug delivery device of the present aspect is configured to receive a fluid in a channel in contact with an electrode. Thereby, the fluid may undergo a charge transfer interaction with the surface of the electrode. The electrode can be a double-sided piece of electrode material. That is, the electrode can be formed of a piece of material having at least two sides (such as elongate sides) or surfaces on which charge transfer interactions can occur. The two sides can be opposite sides of a generally planar piece of material. For example, the two sides can be the top side and the bottom side of a planar piece of material. The shape of the channel in the device can be such that the fluid comes into contact with at least two sides of the electrode. The active surface area of the piece of electrode material in the device is thus advantageously increased, compared with a setup in which only one side of the electrode is active and in contact with the channel. This can improve the efficiency of the device, in that a greater rate of charge transfer is achieved for a given volume of electrode material. This may also reduce the size and/or cost of manufacture of a drug delivery device, in that for a given output a smaller volume of electrode material is required. The piece of electrode material may have further sides which are in contact with the channel. Also, the electrode may be formed of several pieces of electrode material, each of which has two or more sides in contact with the channel.

The drug delivery device of the sixth aspect may be suitable for iontophoretic drug delivery driven by charge supply to the fluid, in the same way as the drug delivery device of the first aspect or the drug delivery device of the fourth aspect.

Preferably, the channel is shaped so as to be in contact with two opposite sides of the double-sided piece of electrode material. The channel may therefore conform to the shape of the electrode material surface so as to advantageously maximise the surface area of the electrode in contact with the channel in which fluid is received for charge transfer.

Preferably, the channel is configured to receive a flow of the fluid, and the channel is shaped such that a bulk flow direction of the fluid changes as the fluid flows past different sides of the double-sided piece of electrode material. The flow path of the fluid may therefore conform to the shape of the electrode surface and "wrap around" the electrode so as to advantageously maximise the surface area of the electrode in contact with the flow path of the fluid in the channel. This can maximise the amount of charge transfer occurring between the electrode and the flowing fluid for a given volume of electrode material. In one example, the direction of flow over one side of the electrode is substantially opposite to the direction of flow over the other side of the electrode.

A seventh aspect of the invention is a method to generate a charged fluid, said method comprising: providing an electrode that is formed of a double-sided piece of electrode material; and flowing a fluid past the electrode, such that the flowing fluid comes into contact with two sides of the piece of electrode material and thereby receives a charge.

An eighth aspect of the invention is a fluid electrode comprising a cavity configured to continuously receive a charged fluid, wherein in use the charged fluid in the cavity generates a continuous electric field that emanates from the cavity.

A ninth aspect of the invention is a method of generating a continuous electric field, the method comprising providing a fluid with a charge to generate a charged fluid, and continuously providing the charged fluid to a cavity in a device, such that the continuous electric field emanates from said cavity.

Any of the above aspects may incorporate features described herein in relation to any other of the aspects.

Brief description of drawings

Figure 1 depicts a traditional iontophoretic drug delivery device.

Figure 2 depicts a drug delivery device according to an embodiment.

Figure 3 depicts a charge-transfer interaction at an electrode that forms a charged fluid to be received in the drug delivery device of Figure 2.

Figure 4 depicts a cavity for continuously receiving a charged fluid, in a drug delivery device according to an embodiment.

Figure 5 depicts a drug channel of a drug delivery device according to an embodiment.

Figure 6 depicts a drug delivery device of a preferred embodiment.

Figure 7 depicts a particular arrangement of a drug delivery device according to an embodiment.

Figure 8 depicts a drug delivery system comprising a drug delivery device implanted in a patient.

Figure 9 depicts a 3D model of a particular construction of a drug delivery device according to an embodiment.

Figure 10 depicts a particular positioning of an electrode and membranes in a drug delivery device according to an embodiment.

Figure 11 depicts another particular positioning of an electrode and membranes in a drug delivery device according to an embodiment.

Figure 12 depicts a drug delivery device comprising an additional electrode according to an embodiment.

Figure 13 depicts a drug delivery device having a channel in contact with two sides of a double-sided electrode, according to an embodiment.

Detailed description of embodiments

Exemplary embodiments will hereinafter be described with reference to the drawings.

The invention provides a novel way of local drug delivery into drug delivery sites such as brain tumours, for example for the treatment of cancer. Devices of the invention may be implanted directly into patient tissue such as directly into the brain tumour or surrounding tissue, and can deliver drugs directly to the delivery site.

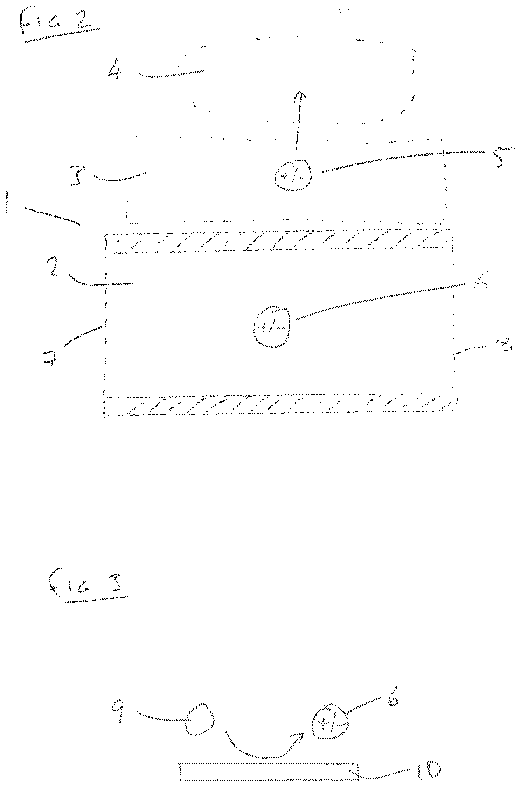

Figure 2 shows a schematic of a drug delivery device 1 according to an embodiment. The drug delivery device includes a cavity 2 configured to continuously receive a charged fluid. In use, a charged particle 5 comprising a drug is received in a space 3 adjacent to the cavity 2. The composition of the charged fluid is described further below, but in general the charged fluid comprises at least one charged component 6 such that the charged fluid in the cavity 2 has a net charge and thus generates an electric field which emanates from the cavity 2. The action of said electric field causes movement of the drug 5 from the space 3 towards a drug delivery site 4 in a bodily tissue.

In use, the drug delivery device 1 may be implanted in the bodily tissue close to the drug delivery site

4. The drug delivery site 4 may be a tumour.

The charged fluid is a fluid (e.g. a liquid) having a formulation which comprises at least one charged component 6, giving the fluid as a whole a net positive or negative charge. For instance, the charged fluid may be a solution of a charged particle (such as an ion) within a neutral solvent (such as water). The charged fluid may comprise any number of different charged components, as long as the fluid as a whole possesses a net charge. Preferably the charged fluid is biocompatible.

The cavity 2 is configured to continuously receive the charged fluid. The cavity 2 may be a channel that defines a flow path for continuous flow of the charged fluid. The channel may comprise an inlet 7 to continuously receive the charged fluid into the cavity, and may comprise an outlet 8 to continuously expel the charged fluid out of the cavity. The continuously-received charged fluid generates a continuous electric field whose action causes the charged particle 5 comprising the drug in the space 3 adjacent to the cavity 2 to move towards the drug delivery site 4, as described in more detail below. The continuously-replenished charged fluid in the cavity may be regarded as a "fluid electrode", that is not subject to the usual problems of electrode degradation in traditional iontophoretic devices.

The charged fluid that is received in the cavity 2 of the drug delivery device 1 may have become charged by a charge transfer interaction of an initial "feed" fluid with an electrode. That is, a feed fluid having no overall charge or an overall charge different to that of the charged fluid, may be brought into contact with an electrode, such that at least one component of the feed fluid interacts with the electrode to gain or lose an amount of charge. Figure 3 shows schematically an interaction of a component 9 present in the feed fluid with an electrode 10 which is a solid electrode, so as to generate the charged component 6 of the charged fluid. As a result of the charge transfer interaction, the net charge of the feed fluid changes, so that the feed fluid becomes the charged fluid comprising the charged component 9.

The charge transfer interaction between the component 9 of the feed fluid and the electrode 10 may be an electrochemical reaction such as oxidation or reduction, that occurs on the surface of the electrode 10. The interacting component 9 of the feed fluid may therefore be a species (referred to herein as a "redox species") that is able to be oxidised or reduced. Preferably, the feed fluid comprises a redox species 9 whose reaction at the electrode surface has a large exchange current density, meaning that the redox species can be easily oxidised or reduced. As a result of the oxidation or reduction of the redox species 9 in the feed fluid, the charge of the redox species 9 changes so that the redox species 9 becomes the charged component 6, thereby causing the net charge of the feed fluid to change as it contacts the electrode 10, and thereby converting the feed fluid into the charged fluid.

Preferably, the redox species is biocompatible.

While in the ideal case the redox species will never get in contact with the patient, it might not always be possible to be avoided completely, and so biocompatibility provides an advantage.

Preferably, the component 9 of the feed fluid which interacts with the electrode 10 to form the charged component 6, undergoes this charge-transfer interaction (such as oxidation or reduction when the component 9 is a redox species) without causing significant generation of a gas at the electrode. Most preferably, the interaction causes no generation of gas at all at the electrode. The driving mechanism of the drug delivery device is thus a charge-transfer interaction that does not cause build-up inside the device or inside a patient in use, which would otherwise damage the device or reduce its efficiency, or cause harm to the patient. The drug delivery device 1 is thus suitable for safe long-term drug delivery to a patient tissue.

A preferred redox species is ferrocyanide, which can be oxidised to form ferricyanide by the follow-

$$Fe(CN)_6^{4-} - 1e^- \rightarrow Fe(CN)_6^{3-}$$

ing reaction:

This electrochemical reaction converts electric charge (i.e. electrons) into ions creating an electric field which repulses positively charged drug molecules or positively charged particles comprising drug molecules in the drug channel, and drives them into the patient's tissue. Ferrocyanide is a preferred redox species in the present invention because its oxidation to form ferricyanide does not cause the formation of a gas. This can improve the safety and efficiency of a drug delivery device. Another suitable redox species is ascorbic acid, which is capable of being oxidised.

When the charged fluid is generated from a feed fluid as described above, the generation of the charged fluid from the feed fluid may occur inside the drug delivery device 1, such as inside the cavity 2, or may occur outside the drug delivery device 1. That is, the cavity 2 can be configured to continuously receive the charged fluid by continuous generation of the charged fluid at an electrode 10 inside the cavity, and/or by continuous supply of the charged fluid in an already charged state to the cavity 2 from elsewhere. When the charged fluid is continuously supplied to the cavity in an already charged state, the charged fluid may have been generated at an electrode 10 which is outside the cavity 2, such as outside the drug delivery device 1 entirely.

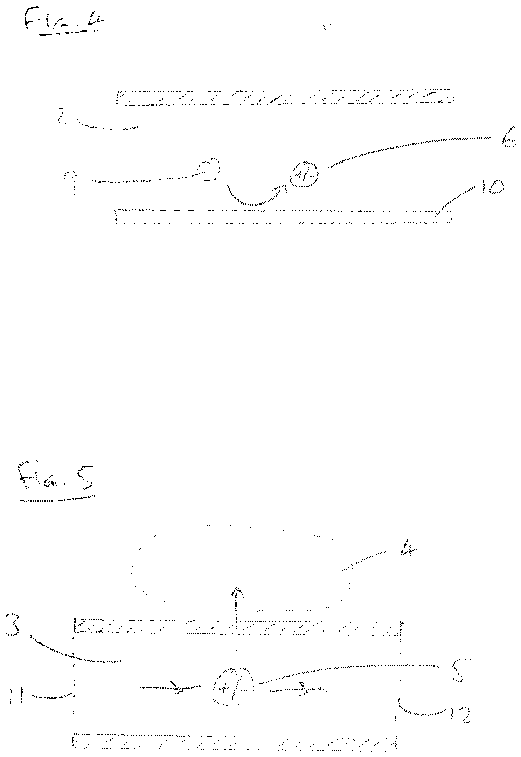

Figure 4 shows the cavity 2 of a drug delivery device. As shown, an electrode 10 is in fluid contact with the cavity 2. The position of the electrode 10 is not particularly limited as discussed further below, but in general the electrode 10 may be positioned inside the cavity 2 itself (e.g. forming a wall of the cavity as shown in Figure 4), or may be positioned outside the cavity 2 yet in contact with a flow path of fluid that subsequently enters the cavity 2 after contact with the electrode 10. The charge transfer between the electrode 10 and the feed fluid (e.g. between the electrode and the redox species 9) occurs continuously such that the charged fluid is continuously generated. The continuous generation of the charged fluid in this way may occur inside the cavity 2 itself (when the electrode is positioned as shown in Figure 4), or may occur in the flow path of fluid preceding the cavity. In the embodiment of Figure 4, the feed fluid is flushed continuously past the electrode 10 in order to continuously replenish the supply of the species (e.g. redox species 9) that undergoes the charge transfer at the electrode 10. The electrode 10 is illustrated as a solid electrode comprised of an electrically conducting solid material, such as a metal.

The drug delivery device 1 can be configured to receive a charged particle 5 comprising a drug, in a space 3 adjacent to the cavity 2, as shown in Figure 2. The charged particle 5 may be a constituent of a drug fluid that is received by the drug delivery device 1. That is, the charged particle 5 comprising the drug may be dissolved or suspended in a drug fluid that also comprises other components, such as a liquid solvent. The composition of the drug fluid is not particularly limited, as long as it comprises the charged particle 5 which itself comprises the drug.

The composition or form of the charged particle 5 itself is also not particularly limited, as long as it carries a charge. The charged particle 5 may itself be a charged drug molecule, such as the chemotherapy drug cisplatin. That is, the charged particle comprising the drug may be the drug itself. Alternatively, the charged particle 5 may comprise one or more drug molecules as well as other components. For instance, the charged particle 5 could be a carrier vehicle of a scale larger than molecular (such as a nanoparticle), which carries one or more drug molecules. In the latter case, the drug molecules themselves do not necessarily need to have a charge, as long as the carrier vehicle has an overall charge. This therefore enables the delivery of uncharged drugs.

A particular application of the present invention is in the delivery of the positively-charged cancer drug cisplatin. Other cancer drugs of any charge can also be delivered. Examples of cancer drugs deliverable by the devices and methods disclosed herein include: cisplatin; carboplatin; oxaliplatin; doxorubicin; gemcitabine; temozolomide; bleomycin; fluorouracil; and irinotecan. The devices and methods disclosed herein are not limited to the delivery of cancer drugs, and it is possible to deliver other charged drug molecules with it targeting different diseases. Examples of other drugs deliverable by the devices and methods disclosed herein include neurotransmitters such as gamma-aminobutyric acid (GABA), and local anaesthetics such as lidocaine. The devices and methods disclosed herein may also be used for delivery of any of the drugs disclosed in 'Use of iontophoresis for the treatment of cancer' (J. D. Byrne, J. J. Yeh and J. M. DeSimone, Journal of Controlled Release 284 (Aug. 2018), pp. 144-151, doi: 10.1016/j.jconrel.2018.06.020) and 'Selected Medicines Used in Iontophoresis' (T. M. Karpinski, Pharmaceutics 2018, 10, 204; doi: 10.3390/pharmaceutics10040204).

The space 3 adjacent to the cavity 2 may be a channel ("drug channel") configured to continuously receive the charged particle 5. For instance, the drug channel may receive a continuous flow of a drug fluid comprising the charged particle 5 which itself comprises the drug. Figure 5 illustrates the drug channel 3 and drug delivery site 4. The drug channel 3 may have a drug channel inlet 11 to continuously receive the charged particle 5 into the drug channel 3. The continuously-received charged particle 5 is continuously driven by action of the electric field from the charged fluid in the cavity, towards the drug delivery site 4. The drug channel 3 may further comprise a drug channel outlet 12 configured to continuously expel from the drug channel the remaining components of the drug fluid after an amount (e.g. all) of the drug has been driven from the drug channel 3 to the drug delivery site 4. Those remaining components may include a solvent, and may include a quantity of the charged particle 5 which has not been driven to the drug delivery site 4.

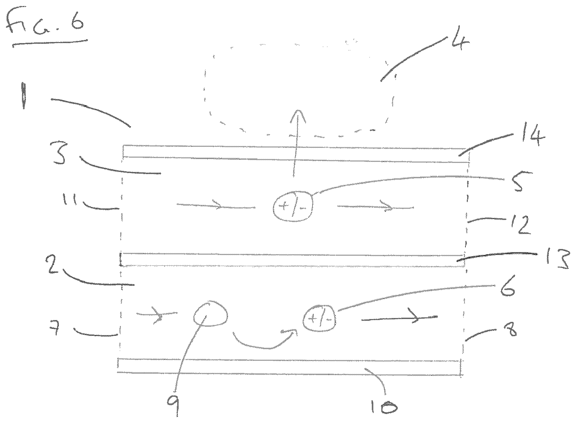

The drug delivery device preferably comprises one or more membranes. A preferred embodiment comprising a first membrane 13 and a second membrane 14 is shown in Figure 6. The properties and function of these membranes are described further below. In the embodiment of Figure 6, the cavity 2 is a channel configured to receive a continuous flow of the charged fluid, an electrode 10 is in fluid contact with the cavity so as to generate the charged fluid (comprising charged component 6) continuously from a feed fluid as described above, and the space 3 adjacent to the cavity is a drug channel configured to receive a continuous flow of the charged particle 5 comprising the drug. However, the provision of membranes such as the first membrane 13 and/or second membrane 14 is also possible in any of the other arrangements of the drug delivery device described herein.

The drug delivery device 1 may comprise a first membrane 13 disposed between the cavity 2 and the space 3 adjacent to the cavity. The first membrane 13 separates the cavity 2 from the space 3 adjacent to the cavity, thereby preventing or reducing mixing of fluids contained in the separate regions. This may advantageously prevent undesired chemical reactions from occurring, such as reactions between drugs contained in the space 3 and the electrode 10, which may otherwise cause the drugs to become useless or even harmful to a patient.

The first membrane 13 may be a porous membrane that is non-selective to the charge polarity of particles that are allowed to cross the first membrane 13. Alternatively, the first membrane 13 may a selective membrane which only allows particles having a certain charge polarity to pass through. For instance, the first membrane 13 may be an ion exchange membrane only allowing a certain polarity of ions to pass through (either an anion exchange membrane only allowing passage of anions, or a cation exchange membrane only allowing passage of cations).

The first membrane 13 may be configured to allow a species having a charge of opposite polarity to that of the charged particle 5 to pass into the cavity from the space adjacent to the cavity. In this embodiment, the movement of such species across the first membrane 13 may enhance the driving of the charged particle 5 comprising the drug towards the drug delivery site 4 by the electric field generated by the charged fluid in the cavity. That is, the action of the electric field generated by the charged fluid in the cavity 2 attracts species (e.g. species present in a drug fluid of which the charged particle 5 is a constituent) having a charge opposite to that of the charged particle 5 to enter the cavity from the space adjacent to the cavity, through the first membrane 13. A charge imbalance is thereby created in the space adjacent to the cavity, driving the movement of the charged particle 5 away from the space and towards the drug delivery site.

The first membrane can be a selective membrane (e.g. an ion exchange membrane) that allows only passage of species having a charge of opposite polarity to that of the charged particle 5, and prevents the passage of species having a charge of the same polarity as that of the charged particle 5. For instance, when the charged particle 5 comprising the drug is positively charged (such as the positively-charged drug molecule cisplatin), the first membrane 13 may be an anion exchange membrane. When the charged particle is negatively charged, the first membrane 13 may be a cation exchange membrane.

The first membrane 13 may be configured to allow a species having a charge of the same polarity to that of the charged particle 5 to pass into the cavity from the space adjacent to the cavity. In a particular embodiment, the first membrane is a selective membrane (e.g. an ion exchange membrane) that allows passage only of species having a charge of the same polarity as that of the charged particle 5, and prevents the passage of species having a charge of opposite polarity to that of the charged particle 5.

The drug delivery device 1 may comprise a second membrane 14 disposed between the space 3 adjacent to the cavity and the drug delivery site 4. The second membrane 13 separates the space 3 from the drug delivery site, thereby preventing or reducing mixing of fluids contained in the separate regions. This may advantageously prevent external fluids from entering the drug delivery device which may harm the efficiency of the device. This may also advantageously prevent bulk fluid in the space 3 (such as a drug fluid) from entering the drug delivery site 4, as described further below.

The second membrane 14 is configured to allow the charged particle 5 comprising the drug to pass to the drug delivery site from the space adjacent to the cavity. The charged particle 5 comprising the drug therefore passes through the second membrane 14 in use of the drug delivery device 1, due to the action of the electric field generated by the charged fluid in the cavity 2.

The second membrane 14 may be configured to allow passage only of the charged particle 5 comprising the drug, while preventing passage of bulk fluid. As described herein, the charged particle 5 comprising the drug may be a constituent of a drug fluid in the space 3 adjacent to the cavity 2. The second membrane 14 may therefore prevent the constituents of that drug fluid other than the charged particle 5 comprising the drug from passing to the drug delivery site 4. In this way, the second membrane advantageously allows the drug delivery device 1 to deliver the drug to the drug delivery site 4 without causing local build-up of pressure at the drug delivery site due to the presence of excess fluid.

The second membrane 14 may be non-selective to the charge polarity of particles allowed to pass through, such as a porous membrane. Alternatively, the second membrane 14 may be a selective membrane (such as an ion exchange membrane) that allows particles only of a certain charge polarity to pass through. For instance, the second membrane 14 may configured to only allow particles having the charge polarity of the charged particle 5 to pass through, while preventing passage of particles of opposite charge. For example, when the charged particle 5 is the positively-charged drug molecule cisplatin, the second membrane 14 may be a cation exchange membrane that prevents the passage of negatively-charged ions.

In a preferred embodiment of the drug delivery device 1, a porous membrane is used as the second membrane 14 to separate the drug channel 3 from the drug delivery site 4 in patient tissue, and an ion exchange membrane is used as the first membrane 13 to separate the cavity 2 from the drug channel 3. However, as described above, it is also possible to use a non-selective porous membrane for the separation of the cavity and drug channel 3 or an ion exchange membrane to separate the drug channel 3 and the drug delivery site 4.

Examples of suitable membranes include Astom Neosepta AMX anion ion exchange membrane (particularly suitable for use as the first membrane 13 separating the drug channel 3 and cavity 2) and Repligen Spectra / Por 3 standard regenerated cellulose membrane 3.5 kD (particularly suitable for use as the second membrane 14 separating the drug channel 3 and the drug delivery site 4).

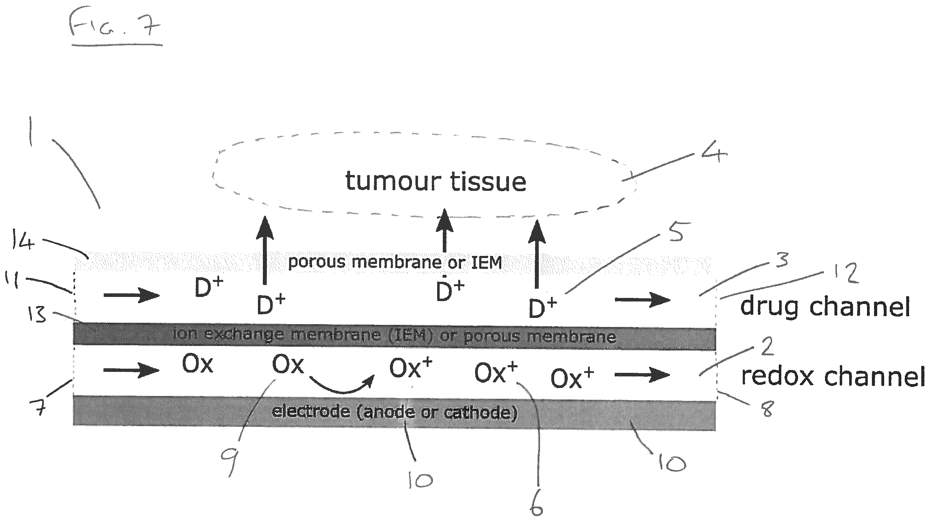

Figure 7 shows a particular arrangement of the drug delivery device 1. The device 1 is essentially a two-channel microfluidic flow cell. Through the first channel called "redox channel" (being an example of the cavity 2), flows an aqueous solution of a redox species 9, being a molecule which can be easily oxidised. The redox channel 2 is in contact with an electrode 10 which may be made of a noble metal (e.g. Pt) or graphite. Adjacent to the redox channel is a drug channel 3, and an aqueous solution of the drug to be delivered flows through the drug channel 3. The redox channel 2 and drug channel 3 are separated from each other by a first membrane 13 that is an anion-exchange membrane. An anion-exchange membrane is a membrane which only allows anions to pass through and which blocks cations. The anion-exchange membrane 13 allows electric current to flow from the electrode 10 through both channels while it prevents the solutions in the two channels from mixing by convection which would have a detrimental effect on device performance. The drug channel 3 is separated from the tissue of the patient by a second membrane 14 which can be, for instance, a porous membrane or an ion exchange membrane. The second membrane 14 prevents pressure-driven flow of the bulk drug solution into the tissue.

In the embodiment of Figure 7, the cavity 2 is a channel ("redox channel"), and contains the charged fluid. The charged fluid comprises positively charged component 6, which gains its charge by oxidation of a redox species 9 at an electrode 10 in fluid contact with the redox channel 2. The redox channel 2 is separate from the drug channel 3 which is a second microfluidic channel containing a drug solution (i.e. a drug fluid that is a solution of a charged drug molecule 5). The redox channel 2 and drug channel 3 are separated by an anion exchange membrane 13 (i.e. the membrane only allows anions to pass and blocks cations). Due to the oxidation of the redox species 9, a positive charge imbalance is created in the redox channel 2, which attracts negatively-charged particles (such as Cl ions) from the drug channel 3. Negatively charged particles from the drug channel 3 move into the redox channel 2. As a result, there is also a positive charge imbalance in the drug channel 3 formed which drives the positively-charged drug molecules 5 (which in this embodiment are cationic drug molecules, $D^+$) through the second (outer) membrane 14 toward the drug delivery site 4 (which is depicted as tumour tissue). The ion-exchange membrane 13 prevents cations to be driven from the redox channel 2 into the drug channel 3 which would compete with the cationic drug 5 (e.g. cancer drug). The drug channel 3 is separated from the tumour tissue 4 by a second membrane 14 that can be a porous membrane or an ion exchange membrane. Continuous flow of the drug solution in the drug channel 3 allows to maintain a large drug concentration in the device 1. The device 1 has four fluidic ports: a redox channel inlet 7, a redox channel outlet 8, and a drug channel inlet 11, and a drug channel outlet 12.

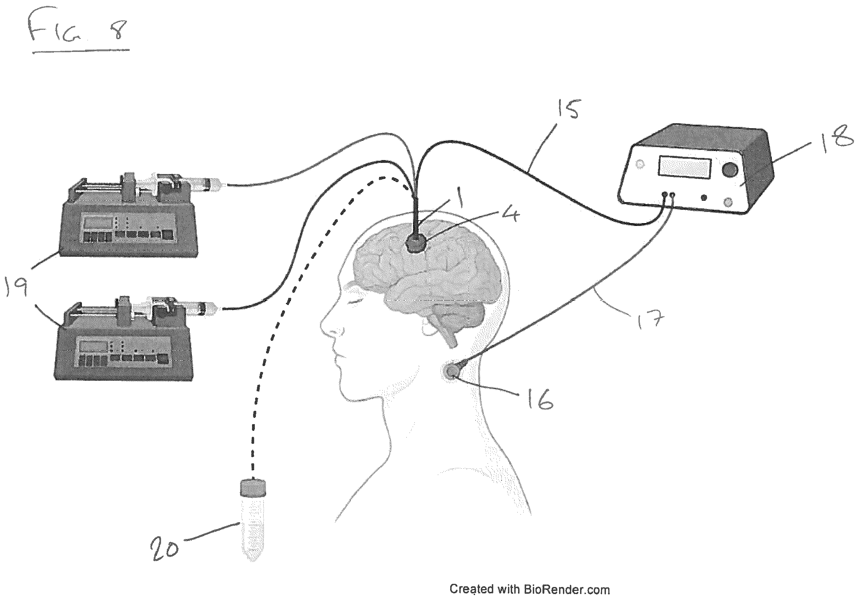

Figure 8 shows an example of how the drug delivery device 1 such as that shown in Figure 7, can be connected to a patient. In this example, the drug delivery site 4 is a brain tumour. An electrode cable 15 may connect the electrode 10 inside the device 1 to a power supply 18. In the arrangement shown, the electrode 10 is inside the device 1, however as described herein the electrode 10 is not limited to being inside the device 1 and could be outside the device 1, such as outside the patient tissue entirely. A counter-electrode 16 may be attached to the patient's skin. The counter-electrode 16 may be a commercial ECG electrode such as an Ag/AgCl electrode. The counter electrode 16 can be connected to power supply 18 by a counter-electrode cable 17. A voltage is applied between the electrode 10 and the counter-electrode 16, which drives the charge-transfer interaction (for instance, the oxidation or reduction of a redox species) to form the charged fluid received in the device 1. The inlets of the redox and drug channels may be connected to a microfluidic flow controller or a syringe driver 19. The outlets of the redox and drug channels can be connected to a waste container 20. The drug delivery device 1 can be implanted into the brain tumour 4 through a cranial window. A commercial port system can be used with the device (e.g. Renishaw Neuroinfuse).

The fluid in the redox channel 2 that gains a charge by interaction (e.g. oxidation or reduction) with the electrode 10 may be known as a "fluid electrode". When the redox channel 2 contains a liquid solution of a redox species, it may be known as a "liquid electrode". The concept of using a fluid electrode such as a liquid electrode has numerous advantages, for instance: it can prevent the formation of corrosive gases which is a major problem with traditional iontophoretic devices using hydrolysis as driving mechanism; it prevents the depletion of the working electrode as it is the case with the 'organic electronic ion pumps' allowing to run the device theoretically forever provided the redox solution is constantly supplied; it allows to separate the drug molecule from the working electrode preventing reactions of the drug molecule on the electrode surface; and it allows to drive the device at high currents, i.e. high drug delivery rates.

An example of a liquid electrode employs the redox species ferrocyanide. When a voltage is applied between the electrode and the counter-electrode, ferrocyanide gets oxidised to ferricyanide, driving the movement of the drug to the drug delivery site as described herein.

The devices and methods disclosed herein are not limited by the charge polarity of the charged particle 5 comprising the drug, or of the charged fluid. While Figure 7 depicts a positively-charged fluid in the redox channel 2 (gaining a positive charge from electrode 10) creating an electric field which drives delivery of a positively-charged drug 5, the principles are equally applicable to negatively-charged fluids and drugs. This would mean inverting the polarity such that the working electrode 10 in the device becomes the cathode instead of the anode and that the sacrificial redox species would be reduced instead of oxidised.

The material from which the electrode 10 is made is not particularly limited. The drug delivery device 1 described herein can operate with any conducting electrode but the reaction rate and therefore the efficiency of the device is dependent on the electrode material. The electrodes can be made of any materials which are commonly used for electrochemical applications. Particular examples of suitable electrode materials include metallic electrodes (such as gold, platinum, platinum-iridium, stainless steel etc) and non-metallic carbon-based electrodes such as graphite.

As discussed herein, the electrode 10 may be inside the drug delivery device 1, such as forming a wall of the cavity 2 as shown in Figure 7. The electrode 10 may also be placed completely outside of the drug delivery device 1, which is advantageous for applications where it is not desired to have the electrode 10 in the patient body. The electrode 10 is generally in fluid contact with the cavity 2, meaning that the charge-transfer interaction that generates the charged fluid may occur inside the cavity itself or at a point on the flow path of the fluid undergoing said interaction that is earlier than the cavity 2.

When the cavity 2 is a channel configured to receive a flow of the charged fluid, the electrode 10 may be placed in said channel at substantially the same point on the flow path of charged fluid as the first membrane 13. Such an arrangement is shown schematically in Figure 7, which depicts the electrode 10 located directly underneath the membrane stack of the first membrane 13 and second membrane 14.

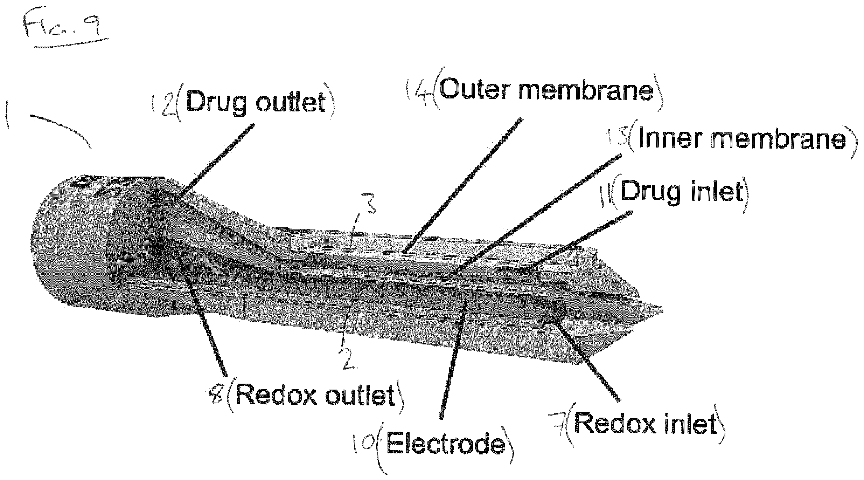

A 3D model of a particular example of the construction of such a drug delivery device is shown in Figure 9.

Figure 9 shows a drug delivery device 1 comprising a cavity 2 which in this embodiment is a channel comprising inlet 7 and outlet 8. Electrode 10 is shown in contact with the channel 2. First membrane 13 is disposed between the channel 2 and drug channel 3, which has a drug channel inlet 11 and drug channel outlet 12. Second membrane 14 is in contact with the drug channel 3. In use, a feed fluid may continuously enter the inlet 7 and become charged by continuous contact with electrode 10 to generate a charged fluid, which constitutes a so-called "fluid electrode". A drug fluid containing a charged particle which comprises a drug, may be introduced continuously to the drug channel 3. By the mechanism described herein, the charged particle can be driven across the second membrane to a drug delivery site outside the drug delivery device 1. The drug delivery device 1 is particularly suitable for implantation in a patient body such as implantation in a brain tumour as shown in Figure 8.

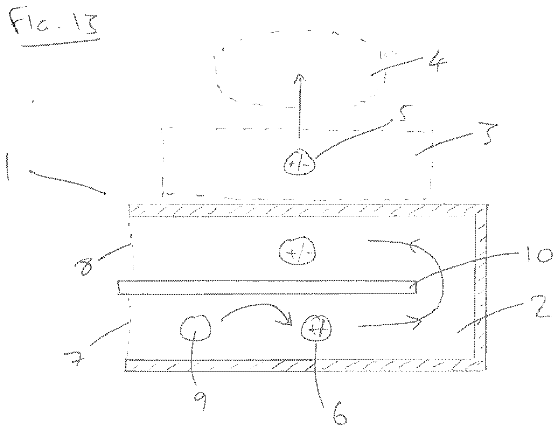

The device shown in figure 9 has a double sided electrode, explained in further detail below with respect to figure 13.

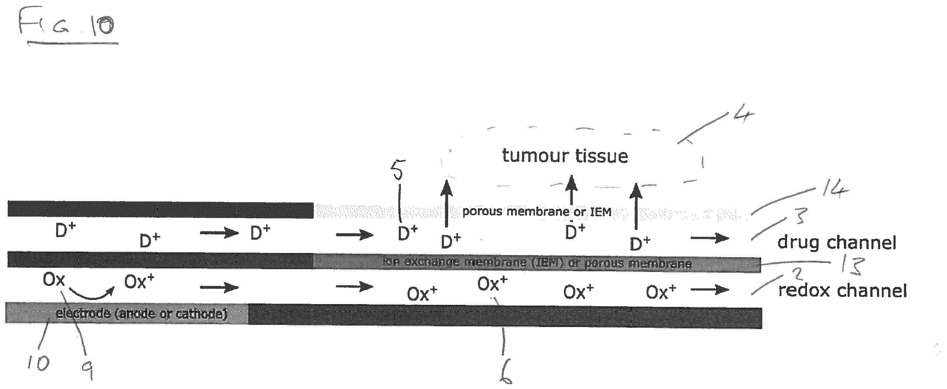

The position of the electrode 10 in relation to the membranes of the drug delivery device 1 in use is however not particularly limited. That is, the electrode 10 does not need to be located directly underneath the membrane stack, and can be shifted in position. Figure 10 shows an alternative arrangement with the electrode being in a different position to that of Figures 7 and 9. In the arrangement of Figure 10, the electrode 10 is placed in a position in the redox channel 2 that is upstream of the position of the first membrane 13, in terms of the flow path of the charged fluid. In this arrangement, the conversion of the feed fluid to the charged fluid by the electrode 10 occurs at a position earlier in the fluid flow path than the position at which the driving of the drug by the electric field occurs. This may give more flexibility to the device geometry in certain applications.

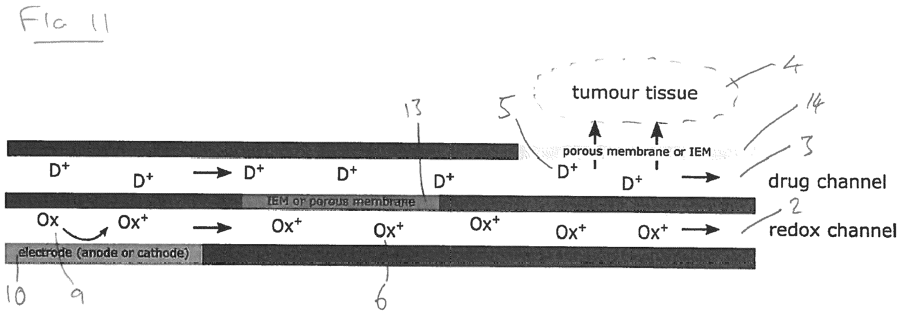

Additionally, also the first membrane 13 separating the redox channel 2 and the drug channel 3 does not need to be located directly underneath the second (outer) membrane 14 or directly above the working electrode 10. Figure 11 shows an arrangement in which the redox channel 2 and drug channel 3 run parallel and adjacent to one another, and the first membrane 13 and second membrane 14 are positioned at different points on the parallel flow paths of the two channels. In the arrangement shown, the movement of particles of opposite charge polarity to the charged particle 5 containing the drug from the drug channel 3 to the redox channel 2 occurs earlier in the flow paths than the movement of the charged particle 5 from the drug channel 3 to the drug delivery site 4. This gives for certain applications more flexibility for the device geometry.

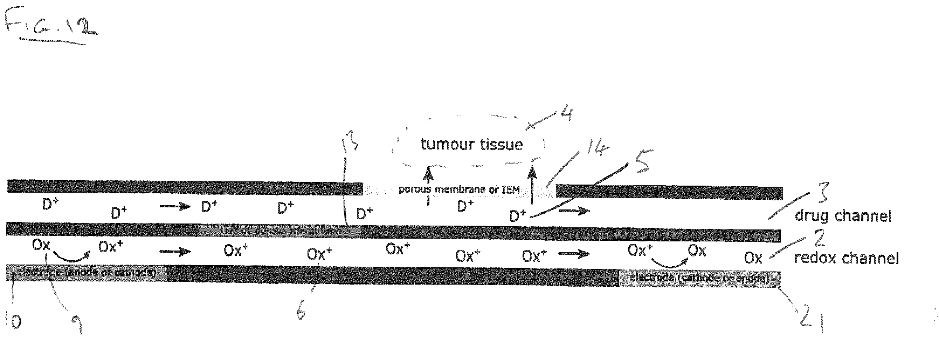

Another different geometry of the drug delivery device 1 is a three-electrode system as shown in Figure 12. In this arrangement, where a second electrode 21 sits in the redox channel 2 which performs the opposite charge-transfer interaction as that of the working ("first") electrode 10, so as to return the charged component 6 of the charged fluid back to its original state before it received a charge from the working electrode. For instance, the second electrode reduces the charged component 6 (such as ferricyanide) back to the redox species 9 (such as ferrocyanide). In the arrangement shown, the redox species 9 in the redox channel 2 gets oxidised on the working electrode 10 (which is an anode), passes the first membrane 13 which separates redox channel 2 and the drug channel 3, and then gets reduced back into its original state on the second electrode 21 (which is a cathode). This allows a circular flow of the redox species (i.e. the redox channel could be a closed system) where the redox species is repeatedly oxidised and reduced. The counter electrode 16 on the skin of the patient will ensure that drug molecules are still delivered. Thus, the fluid constituting the fluid electrode can be continuously recycled so as to minimise wasted material in use of the drug delivery device.

The fluid constituting the "fluid electrode" in the drug delivery device 1 (that is, the fluid that generates an electric field so as to drive the drug to the desired site) may have distinct characteristics that improve the efficiency, efficacy and safety of such devices. Thus, a further embodiment of the invention is a drug delivery device comprising a fluid for delivering a drug to a drug delivery site, the fluid configured to: gain a charge from an electrode; and when charged, generate an electric field to drive a charged particle in a space adjacent to the fluid in a direction away from the fluid, the charged particle comprising the drug. Said drug delivery device may correspond with the drug delivery device 1 of any of the other embodiments described herein. The electrode from which the fluid gains a charge may be the solid, working electrode 10 as described herein.

The fluid gains the charge from the electrode by a charge-transfer interaction of a component in the fluid with the electrode. That is, the fluid comprises a component capable of undergoing a charge-transfer such as a redox reaction, with the electrode, such that the charge of that component changes and the fluid as a whole gains an overall or net charge. Said fluid is preferably configured to gain the charge from the electrode without causing significant generation of a gas at the surface of said electrode. This makes the drug delivery device of this embodiment suitable for long-term efficient use and safe for the patient, because harmful build-up of gases is prevented which would otherwise damage or inhibit the device and cause harm to the patient due to pressure build-up and the possibility of released gases being corrosive or toxic. This property of the fluid also distinguishes it from fluids involved in traditional iontophoretic devices that are absolutely reliant on gas-generating reactions such as hydrolysis to drive the movement of particles such as drugs. Most preferably, the fluid is configured to gain the charge from the electrode without causing any generation of a gas at the electrode.

Suitable examples of the fluid in the drug delivery device are fluids containing ferrocyanide, and fluids containing ascorbic acid. Both of these molecules are capable of being oxidised so as to cause the fluid in which they are contained to become charged. The molecules of the fluid which undergo a charge-transfer interaction to give the fluid a charge may be referred to as "redox species". The drug delivery device may comprise the electrode that is configured to provide the fluid with the charge, or the electrode may be placed outside the drug delivery device such that the charge transfer interaction occurs outside the device. The drug delivery device may be configured to continuously receive a flow of the fluid, and the flow path of the fluid may contact the electrode at a certain point such that the fluid gains the charge continuously as it passes the electrode. Continuously replenishing the fluid constituting the "fluid electrode" ensures that the drug delivery device can continuously deliver a drug over long periods of time because the problem of electrode deterioration that exists in traditional transport devices is overcome.

When the drug delivery device has an electrode in contact with the cavity for generation of the charged fluid, said electrode and cavity may have distinct geometric characteristics that improve the efficiency and efficacy of such devices. Thus, a further embodiment of the invention is a drug delivery device comprising an electrode and a channel, the channel being in contact with the electrode and configured to receive a fluid, wherein: the electrode is formed of a double-sided piece of electrode material; and the channel is shaped so as to be in contact with two sides of the piece of electrode material. This drug delivery device may correspond with the drug delivery device 1 of any of the other embodiments described herein. Said channel corresponds with the cavity 2 of the drug delivery device 1 as described in previous embodiments. A drug delivery device in accordance with this embodiment is shown schematically in figure 13 and also in figure 9.

Figure 13 depicts a drug delivery device 1 including a channel 2 configured to receive a fluid, and an electrode 10 in contact with the cavity 2. The fluid received in the channel 2 is a fluid comprising a component 9 able to interact with electrode 10 to generate a charged component 6. The electrode 10 in this embodiment is formed of a double-sided piece of electrode material. The electrode material is not particularly limited and could be metallic (such as gold, platinum, platinum-iridium, stainless steel etc) or a non-metallic carbon-based electrode material such as graphite. The piece is double-sided, meaning it has at least two active sides or surfaces on which charge-transfer interactions with the fluid can take place. This arrangement advantageously maximises the active surface area of an electrode for a given volume of electrode material. This is distinct from an arrangement in which an electrode is positioned such that one side of the electrode is a surface in contact with the channel and able to undergo charge-transfer interactions, and the other side (e.g. the opposite side) is in contact with the casing of the drug delivery device. In the example depicted in Figure 13, the piece of electrode material constituting the electrode 10 has two elongate sides both in contact with the channel 2, and the channel 2 is shaped such that fluid therein flows past both elongate sides of the electrode 10.

Maximising the active surface area of the electrode 10 in this way in turn maximises the chance of a particular component 9 in the fluid coming into contact with electrode 10 and becoming a charged component 6, thereby maximising the amount of charge that the fluid as a whole gains as it flows through the channel 2. As a result, when the charge of the charged fluid is used to drive movement of charged particles 5 (such as drug particles) in a space 3 adjacent to the cavity, the strength of the electric field emanating from the fluid in the channel 2 (the "fluid electrode") is maximised so that the charged particles 5 are driven efficiently. The configuration of channel 2 and electrode 10 shown in Figure 13 may therefore be used in combination with the drug delivery device 1 of any of the preceding embodiments described herein.

It is also desirable to design the channel 2 and electrode 10 of such a drug delivery device in a way that efficiently uses space, so that the device itself can be effective yet compact and thus suitable for implantation. Thus, in some arrangements, the channel 2 may be shaped so as to be in contact with two opposite sides of the double-sided piece of electrode material, as is the case in Figure 13. Such an arrangement allows efficient use of space because the channel 2 can essentially surround the electrode 10. In the example of Figure 13, fluid in the channel 2 flows past one side of the electrode 10, then flows past the opposite side of electrode 10 while driving movement of particles 5 in the adjacent space 3. In some arrangements, the channel 2 is configured to receive a flow of the fluid, and the channel is shaped such that a bulk flow direction of the fluid changes as the fluid flows past different sides of the double-sided piece of electrode material. This is also the case in Figure 13. This allows continuous replenishing of the fluid which constitutes the "fluid electrode", and also maximises efficiency of use of electrode material and the use of space, by shaping the channel so as to "wrap around" the surfaces of the electrode 10. A compact yet effective device for generating a charged fluid to deliver a drug may therefore be provided.

The arrangement of the channel 2 and electrode 10 shown in Figure 13 enables efficient use of space and of electrode material for generating a charged fluid. The charged fluid may be used in drug delivery by the mechanisms described herein, for instance by further incorporating one or more membranes such as first membrane 13 and second membrane 14 in the device 1. The charged fluid may also be used for other purposes, having been generated efficiently by the arrangement shown.

In the various embodiments of the drug delivery device 1 described herein, the drug delivery device 1 uses electric fields to deliver charged drugs (being either drug molecules themselves, or carrier particles carrying drugs). This technique can deliver selectively only the drug molecules without any solvent (iontophoresis). This allows to achieve high cancer drug concentrations within the brain tumour without increasing the cranial pressure (creating local over-pressures) which is an important advantage over direct injection of cancer drugs into tumours (convection enhanced delivery). Further advantages of this approach are: that the drug does not come in contact with the electrode and therefore does not undergo electrochemical reactions; that no gaseous reaction side products are produced in the device; and that the device could be operated theoretically infinitely long with continuous supply of the charged fluid (or of the redox species).

The devices described herein are suitable for the delivery of drugs (delivered as drug molecules alone or carried on carrier vehicles) as described above. However, devices having substantially the same arrangement as the drug delivery device 1 may also be configured to deliver any charged particle, not necessarily comprising a drug. That is, the principles of the present invention enable the delivery of any charged molecule, atom or vehicle particle loaded with any substance. The present invention therefore provides, in addition to drug delivery devices, methods of transporting charged species in general and methods of generating charged fluids.

For instance, the present invention also provides a method to transport a charged species, the method comprising: continuously receiving a fluid in a cavity of a transport device, the fluid having or being provided with an electric charge so as to generate an electric field; receiving a charged species in a space adjacent to the cavity; and transporting the charged species away from the space, the charged species being transported by the action of said electric field. The method may be implemented using a device similar to the drug delivery device 1 of any of the embodiments herein. For instance, the charged species may be received continuously in the space adjacent the cavity, which itself may be a channel. The transporting the charged species away from the space may involve movement of the charged species across a second membrane 14, which may be a selective membrane, towards a delivery site. The transport may also involve movement of various species across a first membrane 13, which may be a selective membrane, between the cavity of the transport device and the space adjacent the cavity. It will be appreciated that such a method may include steps analogous to any one of the functional features described herein in relation to drug delivery devices.

The present invention similarly provides a method to transport a charged species, the method comprising: providing a fluid with an electric charge, without generating a gas; positioning the charged species in a space adjacent to the fluid that is provided with the electric charge; and driving the charged species in a direction away from the fluid, by action of an electric field generated by the fluid. The method may also be implemented using devices similar to the drug delivery device 1 of any of the embodiments herein. Thus, it will similarly be appreciated that such a method may include steps analogous to any one of the functional features described herein in relation to drug delivery devices.

The present invention similarly provides a method to generate a charged fluid, the method comprising: providing an electrode that is formed of a double-sided piece of electrode material; and flowing a fluid past the electrode, such that the flowing fluid comes into contact with two sides of the piece of electrode material and thereby receives a charge. Such a method could be implemented using a cavity 2 and electrode 10 as described herein in relation to various embodiments of drug delivery devices 1. Thus, it will be appreciated that said method may include steps analogous to any one of the functional features described herein in relation to drug delivery devices.

The present invention further provides a method of manufacturing a drug delivery device, the method comprising: providing a component having a cavity therein, the cavity configured to continuously receive a charged fluid; providing a first membrane between the cavity and a space adjacent to the cavity; and providing a second membrane in contact with the space adjacent to the cavity, the second membrane being configured to selectively allow a particle comprising a drug to pass out of the space adjacent to the cavity in a direction away from the cavity. The so-manufactured drug delivery device may be the drug delivery device 1 of any of the embodiments described herein, and it will be appreciated that the method of manufacturing the drug delivery device may involve providing or creating any combination of the device features described herein in relation to drug delivery devices.

Also, because the devices and methods described herein are able to create constant DC electric fields in a patient body, the technology has other applications beyond delivery of drugs or other particles. For instance, a "fluid electrode" or "liquid electrode" having any of the properties described or depicted herein may itself form an implant that generates a continuous electric field inside the body. Such an implant could be used for various applications including facilitating growth of neurons in a certain direction, thereby assisting in recovery of nerve damage.

Thus, a further aspect is a fluid electrode comprising a cavity configured to continuously receive a charged fluid, wherein in use the charged fluid in the cavity generates a continuous electric field that emanates from the cavity. The fluid electrode of this aspect may be equivalent to that described herein in relation to the drug delivery device 1, except that it does not require the provision of a charged drug or other particle into a space adjacent to the cavity.

**Claims**

1. A drug delivery device configured to deliver a drug to a drug delivery site in a bodily tissue, said drug delivery device comprising a cavity configured to continuously receive a charged fluid, wherein the drug delivery device is configured to:

   receive a charged particle comprising a drug in a space adjacent to the cavity; and
   drive the charged particle from the space to the drug delivery site, the charged particle being driven by the action of an electric field generated by the charged fluid in the cavity.

2. The drug delivery device of claim 1, wherein the space adjacent to the cavity is a drug channel configured to continuously receive the charged particle.

3. The drug delivery device of claim 1 or claim 2, further comprising an electrode in fluid contact with the cavity, the electrode configured to provide a charge to a fluid so as to generate the charged fluid, optionally wherein the electrode is positioned outside of the bodily tissue during delivery of the charged particle to the drug delivery site.

4. The drug delivery device of any one of claims 1-3, further comprising a first membrane between the cavity and the space adjacent to the cavity, optionally wherein

the first membrane is configured to selectively allow a species having a charge of opposite polarity to that of the charged particle to pass into the cavity from the space adjacent to the cavity; and/or
the first membrane is configured to selectively allow a species having a charge of the same polarity as that of the charged particle to pass into the cavity from the space adjacent to the cavity.

5. The drug delivery device of any one of claims 1-4, further comprising a second membrane between the space adjacent to the cavity and the drug delivery site, wherein the second membrane is configured to selectively allow the charged particle comprising the drug to pass to the drug delivery site from the space adjacent to the cavity.

6. A method to transport a charged species, said method comprising:

   continuously receiving a fluid in a cavity of a transport device, said fluid having or being provided with an electric charge so as to generate an electric field;
   receiving a charged species in a space adjacent to the cavity; and
   transporting the charged species away from the space, the charged species being transported by the action of said electric field.

7. A method of manufacturing a drug delivery device, said method comprising:

   providing a component having a cavity therein, the cavity configured to continuously receive a charged fluid;
   providing a first membrane between the cavity and a space adjacent to the cavity; and
   providing a second membrane in contact with the space adjacent to the cavity, the second membrane being configured to selectively allow a particle comprising a drug to pass out of the space adjacent to the cavity in a direction away from the cavity.

8. A drug delivery device comprising a fluid for delivering a drug to a drug delivery site, the fluid configured to:

   gain a charge from an electrode; and
   when charged, generate an electric field to drive a charged particle in a space adjacent to the fluid in a direction away from the fluid, the charged particle comprising the drug.

9. The drug delivery device of claim 8, wherein the fluid is configured to gain the charge from the electrode without causing significant generation of a gas at the electrode.

10. The drug delivery device of claim 8 or claim 9, wherein

the drug delivery device comprises the electrode that is configured to provide the fluid with the charge; and/or
the drug delivery device is configured to continuously receive a flow of the fluid, wherein a flow path of the fluid contacts the electrode such that the fluid gains the charge when it passes the electrode.

11. A method to transport a charged species, said method comprising:

providing a fluid with an electric charge;
positioning the charged species in a space adjacent to the fluid that is provided with the electric charge; and
driving the charged species in a direction away from the fluid, by action of an electric field generated by the fluid.

12. A drug delivery device comprising an electrode and a channel, the channel being in contact with the electrode and configured to receive a fluid, wherein:

the electrode is formed of a double-sided piece of electrode material; and
the channel is shaped so as to be in contact with the two sides of the piece of electrode material.

13. The drug delivery device of claim 12, wherein the channel is shaped so as to be in contact with two opposite sides of the double-sided piece of electrode material.

14. The drug delivery device of claim 12 or claim 13, wherein the channel is configured to receive a flow of the fluid, and the channel is shaped such that a bulk flow direction of the fluid changes as the fluid flows past different sides of the double-sided piece of electrode material.

15. A method to generate a charged fluid, said method comprising:

providing an electrode that is formed of a double-sided piece of electrode material; and
flowing a fluid past the electrode, such that the flowing fluid comes into contact with two sides of the piece of electrode material and thereby receives a charge.

FIG. 1

working electrode

counter electrode

tissue

100

103

104

101

102

FIG.2

FIG.3

FIG.4

2
9
6
10

+/-

FIG.5

3
11
5
12
4

+/-

FIG. 6

EP 4 368 236 A1

FIG. 7

tumour tissue

porous membrane or IEM

drug channel

$D^+$   $D^+$   $D^+$   $D^+$

ion exchange membrane (IEM) or porous membrane

redox channel

Ox   Ox   Ox$^+$   Ox$^+$   Ox$^+$

electrode (anode or cathode)

FIG. 8

Fig. 9

FIG. 10

EP 4 368 236 A1

FIG 11

tumour tissue

porous membrane or IEM

IEM or porous membrane

drug channel

redox channel

electrode (anode or cathode)

$D^+$ $D^+$ $D^+$ $D^+$ $D^+$ $D^+$ $D^+$

$Ox$ $Ox^+$ $Ox^+$ $Ox^+$ $Ox^+$ $Ox^+$ $Ox^+$

FIG. 12

FIG. 13

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 22 38 6080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X Y A | JP 4 361153 B2 (R & R VENTURES KK) 11 November 2009 (2009-11-11) * Reference is made to the attached automatic translation; paragraphs [0001], [0011] – [0018], [0027], [0046]; figure 3 * | 8-10,12, 13 1-5,7 14 | INV. A61N1/04 A61N1/30 |
| X Y | US 2008/027369 A1 (CARTER DARRICK [US] ET AL) 31 January 2008 (2008-01-31) * paragraphs [0002], [0073] – [0075], [0080], [0081] * | 8-10 1-5,7 | |
| Y | US 2011/306878 A1 (DESIMONE JOSEPH [US] ET AL) 15 December 2011 (2011-12-15) * paragraph [0101]; figure 33; example 17 * | 1-5,7 | |
| A | US 2007/037225 A1 (METZGER STEVEN W [US] ET AL) 15 February 2007 (2007-02-15) * paragraphs [0090] – [0104] * | 1-5,7-10 | TECHNICAL FIELDS SEARCHED (IPC) |
| A | EP 0 182 520 A2 (MEDTRONIC INC [US]) 28 May 1986 (1986-05-28) * the whole document * | 1-5,7-10 | A61N |

-/--

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 6 September 2023 | Aronsson, Fredrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................
& : member of the same patent family, corresponding
document

EPO FORM 1503 03.82 (P04E07)

Europäisches
Patentamt

European
Patent Office

Office européen
des brevets

**PARTIAL EUROPEAN SEARCH REPORT**

Application Number

EP 22 38 6080

## DOCUMENTS CONSIDERED TO BE RELEVANT

| Category | Citation of document with indication, where appropriate, of relevant passages | Relevant to claim |
|---|---|---|
| A | DEEPA SRITHARAN ET AL: "Bubble-free electrokinetic flow with propylene carbonate", ELECTROPHORESIS, VERLAG CHEMIE, HOBOKEN, USA, vol. 36, no. 20, 12 October 2015 (2015-10-12), pages 2622-2629, XP071503410, ISSN: 0173-0835, DOI: 10.1002/ELPS.201400443 * abstract * | 1-5,7-10 |
| X | US 2016/184612 A1 (DESIMONE JOSEPH [US] ET AL) 30 June 2016 (2016-06-30) * paragraphs [0004], [0097] – [0099]; figures 14,15 * | 12-14 |
| A | US 5 425 858 A (FARMER JOSEPH [US]) 20 June 1995 (1995-06-20) * line 20, paragraph 10 – line 31, paragraph 12; figure 3 * | 12-14 |
| A | WO 99/40758 A2 (UNIV TENNESSEE RES CORP [US]; ROTH J REECE [US]) 12 August 1999 (1999-08-12) * page 6, lines 18-25; figure 6 * | 12-14 |
| A | US 2016/271606 A1 (CHAWKE BRIAN [IE] ET AL) 22 September 2016 (2016-09-22) * the whole document * | 12 |
| A | US 2 672 948 A (PENNEY GAYLORD W) 23 March 1954 (1954-03-23) * the whole document * | 12 |

**CLASSIFICATION OF THE APPLICATION (IPC)**

**TECHNICAL FIELDS SEARCHED (IPC)**

EPO FORM 1503 03.82 (P04C10)

2

Europäisches
Patentamt

European
Patent Office

Office européen
des brevets

**INCOMPLETE SEARCH
SHEET C**

```
Claim(s) completely searchable:
      1-5, 7-10, 12-14

Claim(s) not searched:
      6, 11, 15

Reason for the limitation of the search (non-patentable invention(s)):

The application does not meet the requirements of Article 53 (c) EPC,
because the subject-matter of claims 6, 11 and 15 relates to a method for
treatment of the human or animal body by therapy.
Claims 6 and 11 comprise the step of transporting charged particles away
from the (charged) fluid. This step, interpreted in light of the
description, is considered therapeutic in nature and thus renders the
whole methods therapeutic.
Claim 15 comprises the step of flowing a fluid past the electrode and
receives a charge. Also this step, interpreted in light of the
description, is considered therapeutic in nature and thus renders the
whole methods therapeutic.
The applicant's arguments have been considered and it is correct that not
any claim can be considered to encompass a therapeutic step, merely
because it does not explicitly exclude such a step. However, in the
present case there is a step in claims 6, 11 and 15 that is only
disclosed as being performed in order to transport a drug into tissue,
and in order to facilitate growth of neurons respectively. Analogously to
the argumentation that a therapeutic method is not encompassed merely
because one could imagine a therapeutic application for the method, even
if such a therapy is not disclosed in the application, one cannot argue
that a non-therapeutic embodiment is encompassed merely because one could
imagine such an embodiment. There is no disclosure in the present
application of a method carried out in a test environment or any other
non-therapeutic context.
Therefore, the objection against claims 6, 11 and 15 is maintained.
```

Europäisches
Patentamt

European
Patent Office

Office européen
des brevets

**Application Number**

EP 22 38 6080

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
1. claims: 1-5, 7-10

   Drug delivery device with a charged fluid that generates an
   electric field that transports charged particles
                        ---


2. claims: 12-14

   Double-sided electrode
                        ---
```

EP 4 368 236 A1

## ANNEX TO THE EUROPEAN SEARCH REPORT
## ON EUROPEAN PATENT APPLICATION NO.

EP 22 38 6080

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2023

| Patent document cited in search report | | Publication date | Patent family member(s) | | Publication date |
|---|---|---|---|---|---|
| JP 4361153 | B2 | 11-11-2009 | JP | 4361153 B2 | 11-11-2009 |
| | | | JP | 2000229128 A | 22-08-2000 |
| US 2008027369 | A1 | 31-01-2008 | EP | 1965856 A2 | 10-09-2008 |
| | | | JP | 2009522011 A | 11-06-2009 |
| | | | US | 2008027369 A1 | 31-01-2008 |
| | | | WO | 2007079193 A2 | 12-07-2007 |
| US 2011306878 | A1 | 15-12-2011 | AU | 2010217957 A1 | 13-10-2011 |
| | | | BR | PI1013399 A2 | 05-04-2016 |
| | | | CA | 2790324 A1 | 02-09-2010 |
| | | | CA | 3152896 A1 | 02-09-2010 |
| | | | CN | 102421479 A | 18-04-2012 |
| | | | EP | 2401027 A1 | 04-01-2012 |
| | | | ES | 2655714 T3 | 21-02-2018 |
| | | | JP | 5653942 B2 | 14-01-2015 |
| | | | JP | 2012519032 A | 23-08-2012 |
| | | | KR | 20110138357 A | 27-12-2011 |
| | | | PL | 2401027 T3 | 30-04-2018 |
| | | | US | 2011306878 A1 | 15-12-2011 |
| | | | US | 2016022985 A1 | 28-01-2016 |
| | | | US | 2020398049 A1 | 24-12-2020 |
| | | | WO | 2010099321 A1 | 02-09-2010 |
| US 2007037225 | A1 | 15-02-2007 | US | 2007037225 A1 | 15-02-2007 |
| | | | US | 2008241858 A1 | 02-10-2008 |
| EP 0182520 | A2 | 28-05-1986 | CA | 1267937 A | 17-04-1990 |
| | | | EP | 0182520 A2 | 28-05-1986 |
| | | | JP | 2584438 B2 | 26-02-1997 |
| | | | JP | S61149168 A | 07-07-1986 |
| US 2016184612 | A1 | 30-06-2016 | EP | 3236838 A1 | 01-11-2017 |
| | | | ES | 2928503 T3 | 18-11-2022 |
| | | | HU | E060224 T2 | 28-02-2023 |
| | | | PL | 3236838 T3 | 19-12-2022 |
| | | | US | 2016184612 A1 | 30-06-2016 |
| | | | US | 2021086000 A1 | 25-03-2021 |
| | | | US | 2023056047 A1 | 23-02-2023 |
| | | | WO | 2016103190 A1 | 30-06-2016 |
| US 5425858 | A | 20-06-1995 | AT | 382583 T | 15-01-2008 |
| | | | AU | 3194495 A | 21-12-1995 |
| | | | CA | 2190829 A1 | 07-12-1995 |
| | | | EP | 0760805 A1 | 12-03-1997 |
| | | | IL | 113798 A | 14-07-1999 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 38 6080

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2023

| Patent document cited in search report | | | Publication date | Patent family member(s) | | Publication date |
|---|---|---|---|---|---|---|
| | | | | JP | 4162709 B2 | 08-10-2008 |
| | | | | JP | H11505463 A | 21-05-1999 |
| | | | | JP | 2008178875 A | 07-08-2008 |
| | | | | MY | 113171 A | 31-12-2001 |
| | | | | SA | 95160229 B1 | 30-11-2005 |
| | | | | US | 5425858 A | 20-06-1995 |
| | | | | US | 5954937 A | 21-09-1999 |
| | | | | WO | 9532803 A2 | 07-12-1995 |
| | | | | ZA | 954133 B | 19-01-1996 |
| WO 9940758 | A2 | 12-08-1999 | AU | 4067799 A | 23-08-1999 |
| | | | | WO | 9940758 A2 | 12-08-1999 |
| US 2016271606 | A1 | 22-09-2016 | CN | 103764285 A | 30-04-2014 |
| | | | | EP | 2694212 A1 | 12-02-2014 |
| | | | | US | 2014111901 A1 | 24-04-2014 |
| | | | | US | 2016271606 A1 | 22-09-2016 |
| | | | | WO | 2012139041 A1 | 11-10-2012 |
| US 2672948 | A | 23-03-1954 | ES | 212609 A1 | 01-03-1955 |
| | | | | US | 2672948 A | 23-03-1954 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2401027 B1

### Non-patent literature cited in the description

- **J. D. BYRNE et al.** Local iontophoretic administration of cytotoxic therapies to solid tumors. *Science Translational Medicine,* February 2015, vol. 7 (273), 273ra14-273ra14
- **J. D. BYRNE et al.** Iontophoretic device delivery for the localized treatment of pancreatic ductal adenocarcinoma. *Proceedings of the National Academy of Sciences,* February 2016, vol. 113 (8), 2200-2205
- **J. D. BYRNE ; J. J. YEH ; J. M. DESIMONE.** Use of iontophoresis for the treatment of cancer. *Journal of Controlled Release,* August 2018, vol. 284, 144-151
- **T. A. SJÖSTRÖM et al.** A Decade of Iontronic Delivery Devices. *Advanced Materials Technologies,* March 2018, vol. 3 (5), 1700360
- **A. WILLIAMSON et al.** Controlling epileptiform activity with organic electronic ion pumps. *Advanced Materials,* 2015, vol. 27 (20), 3138-3144
- **L. WALDHERR et al.** Targeted Chemotherapy of Glioblastoma Spheroids with an Iontronic Pump. *Advanced Materials Technologies,* April 2021, 2001302
- **T. M. KARPINSKI.** Selected Medicines Used in Iontophoresis. *Pharmaceutics,* 2018, vol. 10, 204